# FASCICULE DE BREVET EUROPEEN

(11) **EP 1 503 733 B1**
(45) Date de publication et mention de la délivrance du brevet: **17.09.2008**
(21) Numéro de dépôt: 03755163.7
(22) Date de dépôt: 12.05.2003
(51) Int. Cl.: A61K 9/10, A61K 31/7048, A61K 31/609, A61K 47/44

(54) **COMPOSITIONS ORALES HUILEUSES ANTIPARASITAIRES**
ORALE ÖLIGE ANTIPARASITÄRE ZUSAMMENSETZUNGEN
OLEAGINOUS ORAL ANTIPARASITIC COMPOSITIONS

(30) Priorité: 14.05.2002 FR 0205899
(43) Date de publication de la demande: 09.02.2005
(73) Titulaire: VIRBAC S.A., F-06510 Carros (FR); Janssen Pharmaceutica NV, 2340 Beerse (BE)
(72) Inventeur: DERRIEU, Guy, 06800 Cagnes-sur-Mer (FR); DELHOM, Nathalie, 06140 Vence (FR); DE SPIEGELEER, Bart, 9000 Gent (BE)
(74) Mandataire: Corizzi, Valérie
(86) Numéro de dépôt international: PCT/FR2003/001432
(87) Numéro de publication internationale: WO 2003/099259

(56) Documents cités:
- WO-A-02/09764
- WO-A-94/08566
- WO-A-95/05812
- FR-A- 2 739 778
- US-A- 5 756 474

## Description

La présente invention est relative à de nouvelles compositions, en particulier des compositions destinées à une administration orale à usage vétérinaire constituées d'un véhicule huileux et d'au moins un composé choisi parmi les lactones macro cycliques, en solution, en association avec au moins un composé choisi parmi le closantel et ses sels, en suspension, ces compositions étant par ailleurs physiquement et chimiquement stables.

Les compositions antiparasitaires vétérinaires doivent être adaptées pour combattre les endoparasites ainsi que les ectoparasites pathogènes que l'on rencontre chez les animaux d'élevage et de rapport, mais aussi chez les animaux de zoo, les animaux de laboratoire, les animaux pour l'expérimentation et les animaux de compagnie. On peut citer à titre d'exemples particuliers les bovins, les équins, les ovins, les caprins, les porcins, ou encore les camélidés, les ânes, les daims, les rennes, les buffles, les oiseaux de basse cour, les autruches, les animaux de compagnie chiens ou chats.

Les parasites pathogènes incluent les Plathelminthes tels que les Cestodes et les Trématodes, les Némathelminthes tels que les Nématodes et les Acantocephales, ou encore les Arthropodes. Ces parasites sont très largement répandus dans le règne animal. Le développement parasitaire nécessite le passage par différents stades, oeuf - larves - immatures - adultes, et par la pénétration dans différents tissus des hôtes entraînant des symptômes cliniques de la maladie parasitaire. Aussi bien pendant les phases cliniques que sub-cliniques, ces infestations parasitaires créent des problèmes considérables surtout sur les jeunes animaux qui sont souvent immuno-deficients, sans oublier la possible transmission des parasites à l'homme. Il est donc nécessaire de disposer de traitements pour soigner les animaux parasités mais aussi pour prévenir les infestations des animaux sains. Toutes ces maladies ont des conséquences zootechniques négatives sur les animaux de production, telles que réduction des rendements de carcasse, réduction de la production lactée, des oeufs, laines, peaux et cuirs, et elles entraînent parfois des pertes d'animaux.

Les principes actifs antiparasitaires existants ont des activités différentes vis-à-vis des parasites envisagés. Aussi, il est souhaitable de disposer de compositions associant différents principes actifs efficaces contre différents parasites pouvant infester un même animal, de manière à élargir le spectre d'activité de la composition par rapport à une composition qui ne contiendrait qu'un seul principe actif. Il est par exemple intéressant de pouvoir disposer d'une composition orale associant au moins un composé à action nématodicide à au moins un composé à action trématodicide dans le cas d'infestations concomitantes de l'animal par des parasites pathogènes nématodes (par exemple du genre *Cooperia* spp. ou *Haemonchus* spp. ou *Dictyocaulus* spp.) et des douves (par exemple *Fasciola hepatica*). Les compositions sont alors actives contre toutes ou contre différentes étapes du développement d'un grand nombre de parasites. Par ailleurs, les spectres d'action individuels de chacun des principes actifs associés peuvent se superposer, permettant ainsi à la composition d'être plus efficace dans le traitement de parasites sensibles à la fois aux différents principes actifs. En combattant les endoparasites et les ectoparasites pathogènes, l'intention est de prévenir ou de réduire la maladie parasitaire, la mortalité et les réductions de rendement (par exemple dans la production de la viande, de lait, de laine, d'oeufs etc.), de sorte que l'utilisation des compositions envisagées permette une gestion plus simple et économique des animaux. Il est cependant important que les compositions soient d'une utilisation pratique pour la personne qui administre le traitement.

A ce titre, les compositions orales buvables sont particulièrement adaptées à l'administration de traitements médicamenteux, en particulier chez les moutons et les bovins. Une telle composition doit être stable, facile à conserver, à transporter et à administrer aux animaux, en particulier elle doit être bien acceptée par l'animal.

Ainsi existe-t-il dans le domaine vétérinaire un besoin en traitements efficaces ayant un large spectre d'activité, qui soient pratiques à manipuler et à administrer à l'animal, qui soient bien acceptés par l'animal et qui aient une stabilité suffisante pour permettre une bonne conservation de la composition pendant une durée d'au moins deux ans.

Dans ce contexte, il est très intéressant de disposer d'une composition associant une lactone macro cyclique telle qu'une avermectine (comme l'Ivermectine par exemple) dont les propriétés nématodicides (ou antiparasitaire) sont depuis longtemps très largement utilisées dans le domaine vétérinaire et le closantel ou l'un de ses sels dont les propriétés douvicides, de par l'activité contre les Trématodes, sont aussi très largement utilisées dans le domaine vétérinaire. Cette composition doit pouvoir être facilement administrée aux animaux par voie orale et être stable.

Il est aussi important que la composition orale soit bien tolérée par l'animal et ne soit pas toxique pour l'animal, pour la personne qui administre le traitement ni pour l'environnement. Ainsi, on cherchera à diminuer autant que possible les doses de principes actifs administrées, tout en conservant une efficacité satisfaisante, de manière à ce que la sécurité du traitement pour l'animal soit importante et que l'impact sur l'environnement soit le plus faible possible. On sait en effet que des principes actifs antiparasitaires peuvent être excrétés dans l'environnement, soit à l'état natif, soit sous forme de produits de dégradation issus du métabolisme de l'animal traité. Par exemple, les faeces des bovins en pâturage peuvent être dispersés dans les patures et les cultures et être à l'origine de la destruction d'organismes utiles se trouvant dans les sols. Pour un actif donné, les quantités du principe et/ou de ses métabolites excrétés seront généralement d'autant plus importantes que la dose administrée à l'animal sera élevée.

Il est connu que les lactones macro cycliques, comme l'Ivermectine par exemple, ont des effets négatifs sur l'environnement compte tenu principalement de l'excrétion fécale de cette famille de molécules et de ses métabolites, de son pouvoir antiparasitaire élevé et de sa forte persistance d'activité dans l'organisme d'un animal traité. Beaucoup d'organismes, voire de microorganismes mais surtout les insectes vivant dans le sol peuvent être détruits par ces composés pendant de longues périodes. Il est donc très important de veiller à ce que les quantités de ces composés ou de leurs métabolites excrétés dans l'environnement soient les plus faibles possibles. Il est par ailleurs connu que le closantel ou ses sels, très largement utilisés dans le domaine vétérinaire, sont excrétés de manière importante dans les faeces. Aussi, une composition contenant les deux types de principes actifs sera susceptible de présenter *a priori* un impact négatif sur l'environnement.

Aussi, existe-t-il dans le domaine vétérinaire un besoin en traitements efficaces ayant un large spectre d'activité, et une sécurité d'emploi accrue vis-à-vis de l'animal et de l'environnement qu'il faut satisfaire.

Les avermectines peuvent être administrées par voie orale ou parentérale aux mammifères. Des formulations aqueuses ou organiques à caractère hydrophile sont souvent employées pour administrer les avermectines, Par ailleurs, il est connu qu'elles sont généralement instables et peu solubles, en particulier dans l'eau. Cependant, pour une administration orale d'un médicament, les formulations aqueuses sont généralement préférées car elles ont meilleur goût, sont mieux acceptées par les animaux et sont moins coûteuses que les formulations non aqueuses. Aussi, des efforts importants ont été réalisés pour dissoudre les avermectines dans des formulations aqueuses en utilisant par exemple divers surfactants tels que décrits dans le brevet US 4,389,397. Cependant, du fait de l'insolubilité et de l'instabilité des avermectines dans les formulations aqueuses, les formulations non aqueuses restent désirées. Néanmoins, les formulations non aqueuses qui ont été développées à ce jour pour les administrations parentérales ne conviennent généralement pas à l'administration orale. Par exemple, le brevet US 4,853,372 enseigne des formulations comprenant de l'ivermectine dissoute dans un véhicule à base de propylène glycol et de glycérol formal. De telles compositions peuvent causer des irritations sévères aux animaux si elles sont administrées par voie orale car le propylène glycol est connu pour être responsable d'ataxie et d'hémoglobinurie chez certains animaux tels que le mouton.

La solubilité de l'ivermectine dans des solvants organiques est variable et dépend du solvant utilisé.

Le brevet WO 97/26895 enseigne par exemple que les avermectines ont une solubilité satisfaisante dans la N-methylpyrrolidone ou la 2-pyrolidone et leurs mélanges. Ce sont des solvants convenables pour des formulations d'ivermectine destinées à une administration par injection intramusculaire, injection sous-cutanée, application topique, intubation stomacale et administration orale buvable. Le document présente en particulier des exemples de formulations pour administration orale comprenant en tant que véhicule 5% de N-méthylpyrrolidone, 20% de propylène glycol, 10% de polysorbate 80, 1.5% d'alcool benzylique et le restant d'eau (soit plus de 50%v/v d'eau). De telles compositions sont présentées comme ayant une stabilité améliorée. Cependant, aucune mesure quantitative de la stabilité n'est fournie.

Le brevet US 5,756,454 enseigne des compositions buvables non aqueuses contenant des composés avermectines et contenant de 30 à 45% d'une huile ainsi que de 50 à 70% d'un ester d'acide gras dans lequel est d'abord dissout le composé avermectine. L'ester d'acide gras augmente la solubilité du composé avermectine dans la composition et permet d'ajuster la viscosité. La composition contient en outre un antioxydant soluble dans l'huile (0.01 à 1%) qui a pour effet de prévenir la dégradation du principe actif et d'améliorer la stabilité de la formulation. Aucune mesure quantitative de la stabilité de la composition n'est néanmoins indiquée.

La Demande Internationale WO 02/04764 décrit des compositions comprenant un actif hydrophile et un actif lipophile dans un support huileux ou eau/huile, comprenant éventuellement des solvants. L'actif hydrophile est dispersé dans la composition à l'aide de tensio-actifs.

La demande de brevet EP 0 617 892 décrit la combinaison d'un antibiotique et d'un anthelmintique comme antiparasitaire. L'activité observée pour cette association est suffisamment forte pour permettre la réduction de la quantité totale de produits chimiques employés. De telles compositions sont présentées comme étant stables pendant de nombreuses années. L'antibiotique est préférentiellement l'ivermectine et l'anthelmintique est préférentiellement le closantel. Ce document donne des exemples de composés en milieu organique ou aqueux pouvant être administrés par voie orale. Il mentionne aussi la formulation sous forme d'onguents, ce qui suppose la présence de corps gras et il indique une stabilité possible des compositions pendant plusieurs années.

La très importante variation inter animale par administration d'ivermectine a été mise en évidence par S.E.MARRINER et al., (J.Vet.Pharmacol. Ther., 10 : 175-179) et confirmée par Q.A.McKELLAR et al., (Vet.Parasitology, 39, 1 / 2, 123-136, 1991).

La demanderesse a montré que de manière inattendue une composition à base d'une lactone macro cyclique et de closantel et/ou ses sels dans un véhicule huileux dans lequel la lactone macro cyclique est en solution et le closantel et/ou ses sels est en suspension présente, d'une part, une bonne acceptabilité par les animaux quand elle est administrée par voie orale, d'autre part, est dotée d'une stabilité à la fois physique et chimique supérieure à celle d'une composition huileuse dans laquelle le closantel et/ou ses sels est en solution. Enfin, une telle composition présente une biodisponibilité améliorée de la lactone macro cyclique comparativement à la biodisponibilité de la même lactone macro cyclique lorsqu'elle est employée dans des compositions orales dans un solvant organique et/ou aqueux, telles que les compositions décrites dans la littérature, en particulier les compositions orales commerciales utilisées seules dans les mêmes conditions. Elle présente également une biodisponibilité améliorée comparativement à la biodisponibilité de la même lactone macro cyclique lorsqu'elle est formulée dans une composition orale organique en association avec du closantel ou ses sels. En outre, le véhicule huileux est bien accepté par l'animal quand il est administré par voie orale. Ce véhicule huileux permet d'obtenir une biodisponibilité améliorée de la lactone macro cyclique comparativement à la même association de principes actifs dans un solvant organique et l'état de non-dissolution du closantel dans le véhicule huileux améliore la stabilité de la composition et des principes actifs qu'elle renferme.

La biodisponibilité est définie comme la fraction de la quantité de composé actif, absorbée par un organisme à partir d'une composition, qui arrive dans la circulation générale et par la vitesse à laquelle se produit le phénomène. Cette fraction de la dose absorbée, schématisée par une courbe reliant la concentration et la quantité absorbée par volume sanguin en fonction du temps, est définie, entre autres, par deux paramètres : le Cmax, concentration plasmatique maximale, qui représente un pic sur la courbe tracée, et par l'AUC ou "aire sous la courbe" plasmatique. En règle générale, la biodisponibilité, représentée en particulier par la concentration plasmatique maximale, n'est pas une valeur unique mais une valeur s'inscrivant dans un domaine de concentrations. Ce domaine de concentrations est défini pour sa borne inférieure et pour sa borne supérieure, respectivement par la plus petite valeur connue et par la plus grande valeur connue de la concentration plasmatique maximale.

Par biodisponibilité améliorée il faut entre autre comprendre que la valeur de la concentration plasmatique maximal est inscrite dans un domaine de concentrations bien plus étroit que le domaine connu. La moins grande variation du Cmax obtenue pour les compositions selon l'invention par rapport aux compositions de l'art antérieur permet d'affirmer que la biodisponibilité de l'ivermectine dans les compositions selon l'invention est supérieure à celle des compositions de l'art antérieur.

La demanderesse a montré que de manière inattendue une composition à base d'une lactone macro cyclique, telle que l'ivermectine, et de closantel ou l'un de ses sels comme le sel sodique par exemple, dans un véhicule huileux dans lequel la lactone macro cyclique est en solution et le closantel ou l'un de ses sels est en suspension, donnée par voie orale chez le mouton, présente des valeurs de concentration plasmatique maximale de la lactone macrocyclique délimitant un domaine de concentrations compris entre 12,54 ng/ml et 21,61 ng/ml. Ce domaine de concentration s'étend approximativement du simple au double alors qu'il est connu par les données de la littérature que pour des compositions orales en solvant organique, y compris l'eau, il est compris entre 6,4 ng/ml (R.P.GOGOLEWSKI et al., Vet. Parasitology, 1995, 60, 297-302) et 31,66 ng/ml (Q.A.McKELLAR et al., Vet.Parasitology, 39, 1 / 2, 123-136, 1991)., soit une fourchette de valeurs allant de 1 à presque 5 fois la valeur minimale.

L'efficacité d'un traitement à base d'une lactone macro cyclique étant directement dépendante de la biodisponibilité du principe actif qui dépend elle-même d'une façon générale de la quantité de principe actif dans la composition, il est donc possible avec des compositions selon l'invention de réduire la quantité des lactones macro cycliques, composés éco-toxiques, tout en améliorant leur biodisponibilité afin de présenter l'efficacité souhaitée. Ainsi dans le cas de l'ivermectine présentée dans une composition orale huileuse conforme à l'invention, pour garantir l'efficacité, la quantité usuellement conseillée peut être réduite d'au moins 40%.

Aussi, les nouvelles compositions orales décrites, par la biodisponibilité améliorée qu'elles procurent, et par la diminution de la dose administrée de lactone macro cyclique, permettent de diminuer l'effet néfaste sur l'environnement. Ceci représente un avantage aussi bien économique qu'écologique.
Aussi, la présente invention s'est fixée pour objectifs de pouvoir disposer de compositions orales huileuses permettant d'avoir :
- une réduction de la quantité des principes actifs et/ou de leurs métabolites excrétés dans l'environnement tout en maintenant une activité antiparasitaire sur l'ensemble des familles de parasites sensibles aux principes actifs de l'association,
- une stabilité d'au moins 2 ans des compositions orales afin d'en préserver l'efficacité optimale, c'est à dire une conservation des principes actifs, et en particulier du closantel et/ou ses sels, pendant 2 ans d'au moins 90% et préférentiellement d'au moins 95% de la quantité initiale,
- une bonne acceptabilité par les animaux traités.

La présente invention a pour objet des compositions orales huileuses caractérisées en ce qu'elles comprennent :
- un véhicule huileux,
- au moins un composé choisi parmi les lactones macro cycliques, en solution dans le véhicule huileux,
- au moins un composé choisi parmi le closantel ou l'un de ses sels, en suspension dans ledit véhicule huileux.

Les lactones macro cycliques telles que les composés LL-F28249, les milbémycines et les avermectines sont depuis de nombreuses années utilisées très largement dans le domaine vétérinaire pour le traitement des infestations à nématodes et contre les arthropodes.

La famille des composés très actifs LL-F28249 est constituée par des agents endectocides naturels isolés à partir de bains de fermentation *Streptomyces cyaneogriseus subsp. noncyanogenus*. Les brevet U.S. No. 5,106,994 et U.S. No. 5,169,956 décrivent la préparation des composés majeurs et mineurs, LL-F28249.alpha.-.lambda.. La famille des composés LL-F28249 comprend aussi, mais n'est pas limitée par, les semi-synthétiques 23-oxo et 23-imino de LL-F28249.alpha.-.lambda. tel que cela est enseigné dans le brevet U.S. No. 4,916,154. La moxidectine, chimiquement connue comme la 23-(O-methyloxime)-LL-F28249.alpha., est un dérivé 23-imino particulièrement actif. D'autres exemples de dérivés de LL-F28249 inclus dans la présente invention sont les 23-(semicarbazone)-LL-F28249.alpha. et 23-(thiosemicarbazone)-LL-F28249.alpha..

Les milbemycines, aussi connues comme les antibiotiques de la série B-41, sont des lactones macro cycliques naturelles isolées à partir de *Streptomyces hygroscopicus subsp. aureolacrimosus.* Le brevet U.S. No. 3,950,360 enseigne la préparation des antibiotiques macrolides milbemycin.sub..alpha. 1-.alpha.10, milbemycin.sub..beta.1-.beta.3 etc. Il est communément fait référence à ces composés en tant que milbemycine A, milbemycine B, milbemycine D etc., ou antibiotique B-41A1, antibiotique B-41A3, etc.

Les avermectines, aussi connus comme la famille de composés C-076 sont des lactones macro cycliques naturelles produites par *Streptomyces avermitilis*. Le brevet U.S. No. 4,310,519 décrit l'isolation et la préparation des composés majeurs A 1a (avermectin A1a), A2a, B1a et B2a, et des composés mineurs A1b (avermectin A1b), A2b, B1b et B2b. La famille des composés C-076 comprend aussi les dérivés semi-synthétiques tels que les 22,23-dihydroavermectines décrites dans le brevet U.S. No. 4,199,569. Les dérivés semi-synthétiques comprennent, mais ne sont pas limités à l'ivermectine, l'abamectine, la doramectine, l'eprinomectine, la sélamectine.

L'ivermectine (IVM), chimiquement définie comme la 22,23-dihydroavermectin B1 or 22,23-dihydro C-076 B1, est connue pour être un anthelmintique efficace et peu toxique (Campbell, Ivermectin and Abamectin, Springer, N.Y., 1989). IVM a été notamment employée depuis de nombreuses années par voie orale, par injection sous-cutanée ou transdermique pour traiter des infestations notamment par des nématodes chez les animaux et chez l'homme.

Selon un autre mode de réalisation avantageux desdites compositions orales huileuses, les lactones macro cycliques sont avantageusement choisies parmi l'ivermectine, l'abamectine, l'eprinomectine, la doramectine, la selamectine, la milbémycine oxime, la moxidectine.

Le closantel ou 5'-chloro-4'-(4-chloro-.alpha.-cyanobenzyl)-3,5-diiodosalicyl-o-toluidide, et ses sels sont connus pour leur activité sur les trématodes (comme par exemple *Fasciola hepatica*), les Nématodes hématophages (comme par exemple *Haemonchus contortus, Chabertia ovina.*), les stades larvaires d'*Hypoderma* spp. et autres parasites infestant les animaux.

Le véhicule huileux est choisi parmi les huiles minérales, comme par exemple l'huile de paraffine, ou parmi les huiles végétales, comme par exemple l'huile de sésame, ou l'huile de soja, ou l'huile d'arachide, ou l'huile de coprah, ou l'huile de coton, ou parmi les huiles végétales semi-synthétiques obtenues par fractionnement et/ou hydrolyse et/ou estérification des huiles végétales naturelles comme par exemple les diesters de propylène glycol et d'acides gras ou les triglycérides d'acides gras issus de la noix de coco. Le véhicule huileux peut également être constitué d'un mélange de ces huiles.

Selon un mode de réalisation avantageux desdites compositions orales huileuses selon l'invention, le véhicule huileux est choisi parmi les huiles végétales, comme par exemple l'huile de sésame, l'huile de soja, l'huile d'arachide, ou l'huile de coton, ainsi que leurs mélanges. Préférentiellement, le véhicule huileux comprend de l'huile de soja.

Selon un autre mode de réalisation avantageux desdites compositions orales huileuses, elles comprennent du closantel sodique.

De manière avantageuse, la composition orale selon l'invention devra être réalisée avec du closantel ou l'un de ses sels dont la taille des particules est comprise entre 0,01 et 100 µm et préférentiellement entre 0,1 et 20 µm.

Selon un autre mode de réalisation avantageux desdites compositions orales huileuses, elles peuvent comprendre en outre au moins l'un des composés suivants : les agents épaississants tels que l'éthyl ou méthylcellulose, l'hydroxyéthyl ou hydroxypropyl cellulose, l'hydroxypropyl méthylcellulose, le sel sodique ou potassique de la carboxyméthylcellulose, les carbomers, les silices colloïdales et les silicates, les celluloses microcristallines, les alcools polyvinyliques, les povidones, les copolymères des acides acyliques ou métacryliques, les stéarates d'aluminium, les agents anti-mottants, les anti-agrégants, les adsorbants tels que les bentonites, les silices colloïdales, le trisilicate de magnésium, le talc, les phosphates de calcium, les agents solubilisants qui facilitent la dissolution ou préviennent la précipitation des lactones macro cycliques dans le véhicule huileux tels que le monostéarate de glycérol, les huiles de ricin polyoxyéthylénées, les esters polyoxyéthylénés du sorbitol, l'alcool benzylique, la triacétine, le propylène glycol, la glycérine, le glycofurol, le glycérol formal, les agents anti-oxydants tels que la vitamine E et ses dérivés, l'acide ascorbique et ses dérivés, le 2-*tert*-butyl-4-méthoxyphénol, le 2,6-di-*tert*-butyl-4-méthylphénol, le gallate de propyle, les agents chélatants tels que l'acide édétique et ses sels, l'acide citrique et ses sels, agents conservateurs antimicrobiens, antibactériens ou antifongiques tels que l'alcool benzylique, le trichlorobutanol, le phénol, les esters de l'acide p-hydroxybenzoïque.

Selon encore un autre mode de réalisation desdites compositions orales huileuses, celles-ci contiennent :
- entre 0,01 et 0,48 % (en poids par rapport à la composition totale) d'au moins d'une lactone macro cyclique,
- entre 1 et 24 % (en poids par rapport à la composition totale) de closantel ou de l'un de ses sels.
- un véhicule huileux choisi parmi les huiles végétales, semi-synthétiques ou minérales.

Selon un mode particulièrement préféré de réalisation desdites compositions orales huileuses, celles-ci contiennent:
- entre 0,04 et 0,08 % (en poids par rapport à la composition totale) d'avermectines B₁ ou 22,23-dihydroavermectines B₁ (ivermectines),
- environ 4 % (en poids par rapport à la composition totale) de closantel sous sa forme sodique.
- de l'huile de soja.

De manière avantageuse, dans les compositions orales huileuses à activité endo et ectoparasitaire selon l'invention, lorsqu'elles sont administrées à un animal, en général le rapport pondéral entre les lactones macro cycliques et le closantel et/ou ses sels est compris entre 1 : 200 et 1 : 10, et préférentiellement entre 1 : 100 et 1 : 50. Selon encore une autre disposition préférée de l'invention, la composition orale est un médicament.

Selon encore une autre disposition préférée de l'invention, la composition orale est utilisée pour la préparation d'un médicament pour la prévention et/ou le traitement des endo et/ou des ectoparasites.

Les compositions orales selon l'invention pourront être administrées directement ou préalablement diluées extemporanément, entre autres avec de l'eau, aux animaux par la voie orale (sous forme de drench par exemple).

Les compositions orales selon l'invention peuvent comprendre avantageusement d'autres principes actifs médicamenteux en vue d'élargir le spectre d'activité de la composition, d'améliorer l'efficacité antiparasitaire, et/ou de prévenir et/ou traiter d'autres pathologies de l'animal.

Les compositions sont réalisées et conditionnées, de manière connue de l'Homme du métier, afin de préserver l'intégrité des molécules.

L'invention sera mieux comprise à la lecture du complément de description qui suit et qui se réfère à des exemples de réalisation de compositions orales huileuses conformes à l'invention, ainsi que de démonstration de leurs propriétés particulières. Il doit être bien entendu, toutefois, que ces exemples ne sont donnés qu'à titre d'illustrations de l'invention et n'en constituent en aucune manière une limitation.

### EXEMPLE 1

Biodisponibilité de l'ivermectine et du closantel chez le mouton par voie orale et acceptabilité des compositions par les animaux.

### A-1^{er} essai

### a) Mode opératoire :

Chacun des 4 groupes de 6 moutons, chaque mouton d'un poids compris entre 25 et 40 kg, les groupes étant composés de façon homogène de moutons de race européenne et des deux sexes, a été traité par voie orale par l'une des compositions orales suivantes :

On prépare selon les techniques connues de l'homme du métier une composition orale huileuse A, une composition organique B et on utilise des préparations orales de formule C et D du commerce.

Le contenu des compositions A à D est illustré dans le tableau I ci-dessous. Les quantités des composants sont exprimées en pourcentage en poids, gramme, par rapport au volume total, 100 ml, de la composition. Le volume final est obtenu par addition d'huile de soja dans la composition A, par addition d'eau dans la composition B, et par addition de l'excipient pour les compositions commerciales D et C.

La suspension huileuse de formule A est une composition huileuse selon l'invention.

La solution organique B est une composition organique contenant la lactone macro cyclique, et le closantel sous forme de sel sodique, représentant les compositions décrites dans la littérature ou équivalente à celles du commerce. Pour avoir une meilleure stabilité, cette solution est tout d'abord préparée avec un véhicule exclusivement organique dans lequel les concentrations en ivermectine et en closantel sous forme de sel sodique sont multipliées par quatre. Afin d'éviter des irritations sévères lors de l'administration aux animaux elle est diluée avec de l'eau, au quart, au moment de son utilisation.

La composition C correspond à un produit commercial « Oramec^{®} » commercialisée par les Laboratoires MERIAL Animal Health Limited,

La composition D correspond au produit « Flukiver^{®} » commercialisé par Janssen Animal Health BVBA.

**Tableau 1**

| FORMULE | | COMPOSITION | | | |
|---|---|---|---|---|---|
| | | A | B (Diluée) | C | D |
| Ivermectine (22,23-dihydroavermectines B₁ₐ /B_{1b}) | | **0,08** | **0,08** | **0,08**** | **-** |
| Closantel* | | **4.0** | **4,0** | **-** | **5,0** |
| Agent anti-mottant (Silice colloïdale) | | **1,58** | **-** | | |
| Agent anti-oxydant (BHA/BHT) | | **0,03** | **0,0075** | | |
| Agent conservateur (Alcool benzylique) | | **1,00** | **-** | | |
| Polyvinylpyrrolidone | | | **1,0** | | |
| Propylène Glycol | | | **25 ml** | | |
| Eau | q.s. | | **100 ml** | | **20,72** |
| Huile de soja | q.s. | **100 ml** | **-** | **-** | **-** |
| Excipient | q.s. | | **-** | **100 ml** | **100 ml** |

| | | | | | |
|---|---|---|---|---|---|
| *Le Closantel est introduit sous la forme de sel sodique en quantité suffisante pour garantir une teneur en Closantel de 4% (p/V) dans la composition finale. ** L'ivermectine présente dans la formulation commerciale a été dosée et un surdosage de 10% à été mis en évidence par rapport aux quantités annoncées sur l'étiquette du produit commercialisé. Ainsi, il y a 0,088% (p/V) d'ivermectine dans la composition finale. | | | | | |

### b) mode de traitement des groupes :

### b-1) traitement : Chaque animal de chaque groupe reçoit par voie orale en une fois à l'aide d'un pistolet drogueur les volumes suivants :

- groupe A : 1 ml pour 4 kg de poids vif
   (soit 200 µg/kg d'Ivermectine et 10 mg/kg Closantel),
- groupe B : 1 ml pour 4 kg de poids vif
   (soit 200 µg/kg d'Ivermectine et 10 mg/kg Closantel),
- groupe C : 1 ml pour 4 kg de poids vif
   (soit 200 µg/kg d'Ivermectine),et
- groupe D : 1ml pour 5 kg de poids vif
   (soit 10 mg/kg de Closantel),.

### b-2) prélèvements sanguins et analyse:

9 ml de sang ont été prélevés à chaque animal sur tubes d'héparine, avant le traitement et après traitement par l'une des compositions A, B, C, et D à 2, 4, 8 , 12, 24, 48, et 72 heures et ensuite après 7, 14, 21, 28, et 35 jours.

L'analyse des échantillons a été conduite selon des techniques classiques bien connues de l'Homme de métier.

### c) étude comparative de la biodisponibilité des molécules actives :

### c-I) Ivermectine :

Les concentrations plasmatiques (déviation standard), exprimées en ng d'Ivermectine par ml en fonction du temps, sont rassemblées dans le tableau II suivant :

**Tableau II**

| Temps en jours | Concentrations | | |
|---|---|---|---|
| | Composition A | Composition B | Composition C |
| 0.00 | **< LDD** | **< LDD** | **< LDD** |
| 0.08 | **0,20±0,28** | **2,21±3,16** | **4,88±8,72** |
| 0.16 | **3,19±1,82** | **9,11±5,48** | **9,34±10,59** |
| 0.33 | **14,68±4,95** | **16,15±4,70** | **13,24±5,02** |
| 0.50 | **20,19±1,42** | **15,87±4,70** | **14,26±4,13** |
| 1.00 | **16,62±3,18** | **10,65±4,13** | **10,24±4,30** |
| 2.00 | **7,86±2,45** | **4,34±1,44** | **4,08±2,26** |
| 3.00 | **4,44±1,70** | **1,67±0,87** | **1,81±1,10** |
| 7.00 | **0,78±0,58** | **0,30±0,14** | **0,28±0,15** |
| 14.00 | **0,18±0,24** | **0,21±0,21** | **< LDD** |
| 21.00 | **< LDD** | **0,12±0,17** | **< LDD** |
| 28.00 | **0,03±0,01** | **< LDD** | **< LDD** |
| 35.00 | **< LDD** | **< LDD** | **< LDD** |

| | | | |
|---|---|---|---|
| LDD : valeur inférieure à la limite de détection, elle est remplacée par la valeur 0,025 ng/ml qui correspond à LDD/2 pour les calculs et les représentations graphiques | | | |

### c-2) Closantel :

Les concentrations plasmatiques (déviation standard), exprimées en µg de closantel par ml en fonction du temps, sont rassemblées dans le tableau III suivant :

**Tableau III**

| Temps en jours | Concentrations | | |
|---|---|---|---|
| | Composition A | Composition B | Composition D |
| 0.00 | **< LDD** | **< LDD** | **< LDD** |
| 0.08 | **< LDD** | **4,51±6,79** | **0,57±1,27** |
| 0.16 | **4,98±3,32** | **18,62±12,29** | **8,58±4,16** |
| 0.33 | **32,86±15,11** | **43,90±13,04** | **43,06±14,98** |
| 0.50 | **44,21±9,42** | **55,72±9,51** | **51,44±5,60** |
| 1.00 | **63,24±11,54** | **65,11±8,1** | **69,13±7,68** |
| 2.00 | **59,56±10,10** | **60,46±5,53** | **64,22±5,33** |
| 3.00 | **57,99±9,47** | **59,33±6,81** | **60,37±4,92** |
| 7.00 | **47,43±7,68** | **48,63±5,80** | **50,41±3,85** |
| 14.00 | **42,95±9,68** | **43,13±5,90** | **43,93±4,75** |
| 21.00 | **38,11±8,82** | **37,73±5,70** | **38,59±4,82** |
| 28.00 | **30,63±7,44** | **30,13±5,20** | **30,92±3,44** |
| 35.00 | **24,52±6,05** | **26,08±5,06** | **26,44±3,34** |

| | | | |
|---|---|---|---|
| LDD : valeur inférieure à la limite de détection, elle est remplacée par la valeur 0,05 µg/ml qui correspond à LDD/2 pour les calculs et les représentations graphiques | | | |

### B -2^{ème} essai

### a) Mode opératoire :

Chacun des 5 groupes de 6 moutons, chaque mouton d'un poids compris entre 24 et 34 kg, les groupes étant composés de façon homogène de moutons de race européenne et des deux sexes, a été traité par voie orale par l'une des compositions orales suivantes :

On prépare selon les techniques connues de l'homme du métier une composition orale huileuse A, une composition orale huileuse E, une composition orale huileuse F, et on utilise des préparations orales de formule C et D du commerce.

Le contenu des compositions A, C, D, E et F est illustré dans le tableau IV ci-dessous. Les quantités des composants sont exprimées en pourcentage en poids, gramme, par rapport au volume total, 100 ml, de la composition. Le volume final est obtenu par l'huile de soja dans les compositions A, E, F, et l'excipient pour les compositions commerciales C et D.

La suspension huileuse de formule A correspond à une composition huileuse selon l'invention, composition identique à la composition A utilisée dans le premier essai.

La suspension E correspond de façon similaire à la composition huileuse de formule A mais ne contenant que du closantel, sous forme de sel sodique,

La suspension F correspond de façon similaire à la composition huileuse de formule A mais ne contenant que de l'ivermectine,

La composition C correspond à un produit commercial « Oramec^{®} » des Laboratoires MERIAL Animal Health Limited, composition commerciale identique à la composition C utilisée dans le premier essai,

La composition D correspond à un produit commercial « Flukiver^{®} » de Janssen Animal Health BVBA, composition commerciale identique à la composition D utilisée dans le premier essai.

**Tableau IV**

| FORMULE | | COMPOSITION | | | | |
|---|---|---|---|---|---|---|
| | | A | E | F | C | D |
| Ivermectine (22,23-dihydroavermectines B₁ₐ /B_{1b}) | | 0,08 | | 0,08 | 0,08** | - |
| Closantel | | 4,0* | 4,0* | | | 5,0 |
| Agent anti-mottant (Silice colloïdale) | | 1,58 | 1,58 | 1,58 | | |
| Agent anti-oxydant (BHA/BHT) | | 0,03 | 0,03 | 0,03 | | |
| Agent conservateur (Alcool benzylique) | | 1,00 | 1,00 | 1,00 | | |
| Huile de soja | q.s. | 100 ml | 100 ml | 100 ml | | - |
| Excipient | q.s. | | - | | 100 ml | 100 ml |

| | | | | | | |
|---|---|---|---|---|---|---|
| *Le closantel est introduit sous la forme-de sel sodique en quantité suffisante pour garantir une teneur en closantel de 4% (p/V) dans la composition finale. ** L'ivermectine présente dans la formulation commerciale a été dosée et un surdosage de 10% à été mis en évidence par rapport aux quantités annoncées sur l'étiquette du produit commercialisé. Ainsi, il y a 0,088% (p/V) d'ivermectine dans la composition finale. | | | | | | |

### b) mode traitement des groupes :

b-1) traitement : Chaque animal de chaque groupe reçoit par voie orale en une fois à l'aide d'un pistolet drogueur les volumes suivants :
- groupe A : 1 ml pour 4 kg de poids vif
   (soit 200 µg/kg d'ivermectine et 10 mg/kg closantel),
- groupe E : 1 ml pour 4 kg de poids vif
   (soit 10 mg/kg closantel),
- groupe F : 1 ml pour 4 kg de poids vif
   (soit 200 µg/kg d'ivermectine),
- groupe C : 1 ml pour 4 kg de poids vif
   (soit 200 µg/kg d'ivermectine),et
- groupe D : 1ml pour 5 kg de poids vif
   (soit 10 mg/kg de closantel).

### b-2) prélèvements sanguins et analyse:

le mode opératoire est décrit dans le premier essai.

### c) étude comparative de la biodisponibilité des molécules actives :

### c-1) Ivermectine :

Les concentrations plasmatiques (déviation standard), exprimées en ng d'ivermectine par ml en fonction du temps, sont rassemblées dans le tableau V suivant :

**Tableau V**

| Temps en jours | Concentrations | | |
|---|---|---|---|
| | Composition A | Composition F | Composition C |
| 0.00 | 0 | 0 | 0 |
| 0.08 | 7,21±4,58 | 2,04±1,06 | 19,72±20,05 |
| 0.16 | 11,91±5,94 | 6,87±2,68 | 23,03±14,40 |
| 0.33 | 16,36±5,36 | 14,69±2,17 | 22,49±8,48 |
| 0.50 | 16,81±4,27 | 14,99±4,60 | 20,6±7,38 |
| 1.00 | 14,29±4,97 | 12,36±6,32 | 14,5±5,16 |
| 2.00 | 6,91±2,76 | 5,34±3,00 | 7,13±2,31 |
| 3.00 | 3,92±1,30 | 3,17±1,96 | 3,78±1,92 |
| 7.00 | 0,9±0,26 | 0,73±0,44 | 0,87±0,42 |
| 14.00 | 0,21±0,17 | 0,16±0,10 | 0,14±0,10 |
| 21.00 | 0,03±0,07 | 0,04±0,06 | 0,03±0,04 |
| 28.00 | < LDD | < LDD | < LDD |
| 35.00 | < LDD | < LDD | < LDD |

| | | | |
|---|---|---|---|
| LDD : valeur inférieure à la limite de détection, elle est remplacée par la valeur 0,025 ng/ml qui correspond à LDD/2 pour les calculs et les représentations graphiques | | | |

### c-2) Closantel :

Les concentration plasmatiques (déviation standard), exprimées en µg de closantel par ml en fonction du temps, sont rassemblées dans le tableau VI suivant :

**Tableau VI**

| Temps en jours | Concentrations | | |
|---|---|---|---|
| | Composition A | Composition E | Composition D |
| 0.00 | < LDD | < LDD | < LDD |
| 0.08 | 9,34±4,74 | 5,43±8,51 | 6,36±6,27 |
| 0.16 | 25,22±8,65 | 13,63±11,70 | 18,23±7,59 |
| 0.33 | 44,18±10,52 | 28,06±8,48 | 40,54±11,77 |
| 0.50 | 51,62±8,28 | 38,77±7,47 | 47,34±13,15 |
| 1.00 | 59,25±9,10 | 49,7±5,82 | 59,63±8,85 |
| 2.00 | 56,17±6,22 | 50,61±7,66 | 57,33±8,41 |
| 3.00 | 50,28±5,76 | 45,52±6,06 | 54,2±7,65 |
| 7.00 | 41,44±6,52 | 38,66±7,05 | 45,11±7,15 |
| 14.00 | 30,98±7,71 | 30,8±6,56 | 35,31±7,02 |
| 21.00 | 24,62±6,83 | 25,17±6,78 | 30,23±5,17 |
| 28.00 | 19,06±6,17 | 18,93±4,74 | 22,87±4,63 |
| 35.00 | 14,88±5,82 | 15,13±4,43 | 16,74±5,31 |

| | | | |
|---|---|---|---|
| LDD : valeur inférieure à la limite de détection, elle est remplacée par la valeur 0,05 µg/ml qui correspond à LDD/2 pour les calculs et les représentations graphiques | | | |

### C-Résultats :

La biodisponibilité est définie, entre autres, par la quantité de matière(s) active(s) qui arrive dans la circulation, soit par le Cmax (concentration plasmatique maximale) représentée par le sommet du pic sur la courbe, et par l'AUC (aire sous la courbe plasmatique). Pour chaque parasite, nous connaissons le taux minimum nécessaire de la matière active pour atteindre une activité létale sur les parasites cibles. Ainsi les produits de référence, Oramec^{®} pour l'ivermectine et Flukiver^{®} pour le closantel, nous donnent les seuils minimaux à atteindre pour le parasite le moins sensible (dose-limitant).

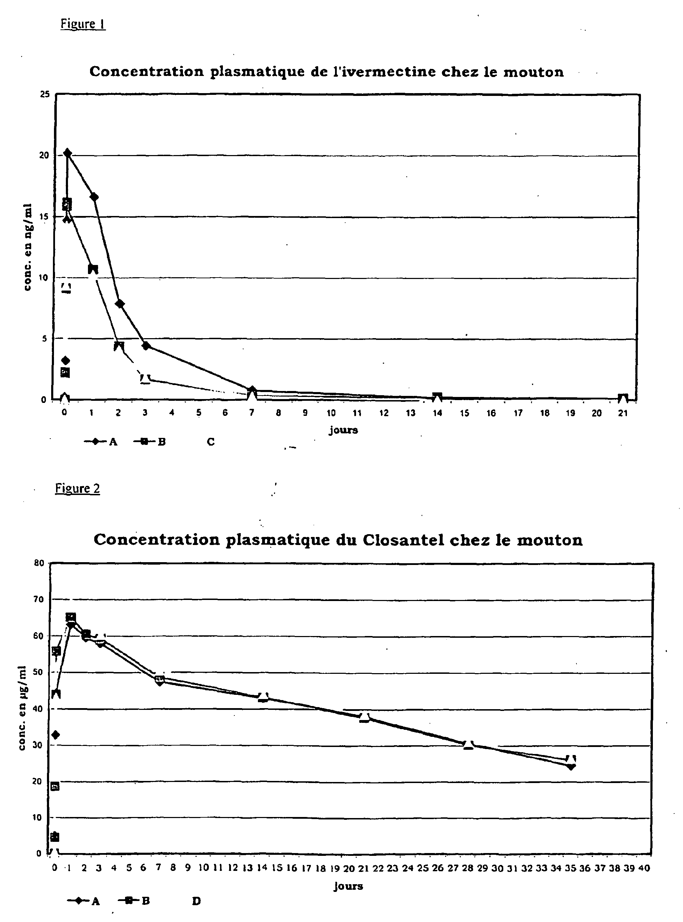

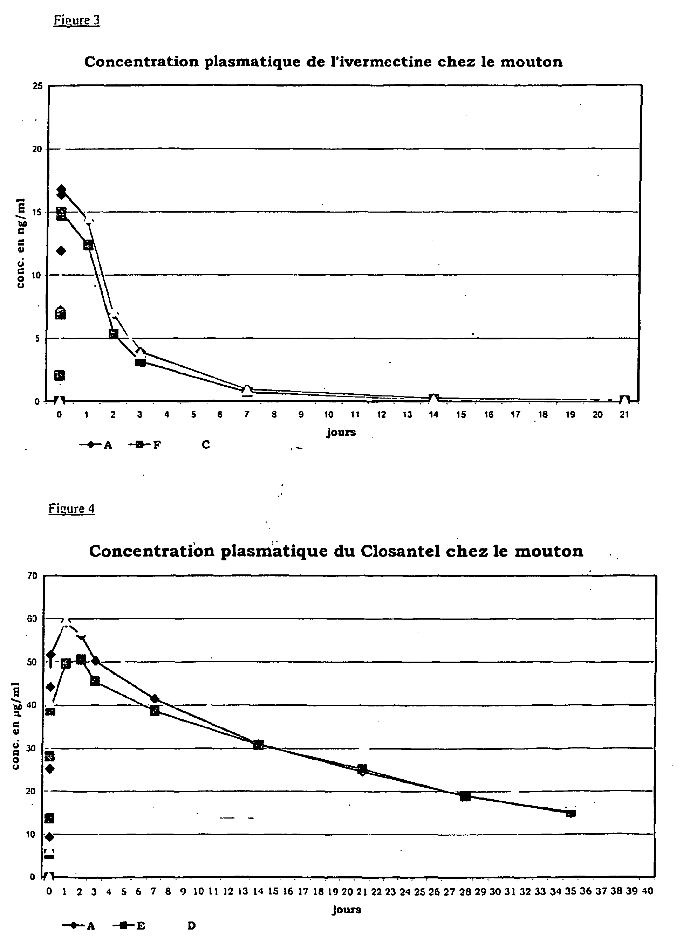

Comme le montrent les figures 1 et 3, données en annexe, la quantité maximale d'ivermectine circulante obtenue (pic plasmatique) avec la composition A selon l'invention est de 21,61 ng/ml (essai 1) et de 12,54 ng/ml (essai 2), et elle est comprise entre 10,13 ng/ml (essai 1) et 27,98 ng/ml (essai 2 à t=0,50j), pour le produit commercial de référence, composition C (les mesures obtenues dans l'essai 2 à t=0,16j et à t=0,30j confirment l'extrême variabilité des concentrations plasmatiques observées pour l'ivermectine dans la composition C).

Ces résultats confirment ce qui est connu dans la littérature pour la composition C, à savoir une très grande variabilité du Cmax. Par contre, les résultats obtenus avec la composition A selon l'invention sont inclus dans un domaine de Cmax plus restreint, même en tenant compte de la variabilité due à un test *in vivo*. On note une augmentation de plus de 19% en comparant la quantité maximale circulante d'ivermectine obtenue dans le deuxième essai avec la composition A selon l'invention à la quantité maximale circulante d'ivermectine obtenue dans le premier essai avec la composition commerciale C. Ces résultats démontrent la meilleure fiabilité de l'administration de l'ivermectine lorsqu'elle est introduite dans des compositions selon l'invention par rapport aux compositions commericales qui ne comprennent que de l'ivermectine. Ainsi, pour obtenir la même quantité maximale circulante d'ivermectine il suffit d'utiliser pour la composition A conforme à l'invention seulement 0,065 % p/p d'ivermectine. Par ailleurs, si l'on tient compte, en outre, du surdosage de 10% en ivermectine dans la formule C on réduit d'au moins 25% la quantité nécessaire de lactone macrocyclique tout en gardant l'activité antiparasitaire recherchée.

Le rapport pondéral entre l'Ivermectine et le Closantel est de 1 : 50 dans la composition A, et peut être ramené à 1:62,5, , tout en gardant l'activité antiparasitaire recherchée de la lactone macro cyclique.

On constate que par rapport à des compositions comprenant de l'ivermectine et du closantel en solution en milieu organique (composition B), les compositions selon l'invention ont une valeur de Cmax et AUC augmentée pour l'ivermectine et conservée pour le closantel.

Comme le montrent les figures 2 et 4, les compositions, quelle que soit leur formulation, donnent des quantités maximales de closantel respectivement très voisines, comprises entre 49,70 et 69,16 µg/ml, ce qui montre par ailleurs une bonne reproductibilité compte tenu de la variabilité due à des tests *in vivo*, réalisés à des moments différents. On en déduit que la composition selon l'invention n'a pas modifié la biodisponibilité, et partant l'efficacité du closantel.

### Acceptabilité par les animaux traités :

### Au cours des essais,

la demanderesse a par ailleurs évalué la prise de composition orale huileuse selon l'invention pour vérifier sa bonne acceptation par l'animal traité comparativement à des compositions orale déjà commercialisées.

Le comportement des animaux a été observé par l'opérateur qui administre les traitements. Aucune différence n'a été relevée en ce qui concerne l'acceptation des compositions buvables par les animaux dans les différents groupes.

Ainsi, la composition orale huileuse selon l'invention est bien accepté et tolérée par les animaux traités, ce qui confirme la facilité d'utilisation pour la personne administrant la composition selon l'invention et la sécurité d'emploi, l'animal ne refusant pas le traitement.

### EXEMPLE 2

Etude comparative de la stabilité de l'ivermectine et du closantel.

On prépare selon les techniques connues de l'homme du métier une composition orale huileuse A selon l'invention se présentant sous la forme d'une suspension, une composition orale huileuse G se présentant sous forme d'une solution et une composition orale en milieu organique B.

Le contenu des compositions A, B et G est illustré dans le tableau VII. Les quantités des composants sont exprimées en pourcentage en poids (gramme) par rapport au volume total (100 ml) de la composition. Les résultats des tests de stabilité sont résumés dans le tableau VIII.

**Tableau VII : Compositions qualitatives et quantitatives des formulations comparées.**

| FORMULE | | COMPOSITIONS | | |
|---|---|---|---|---|
| | | A | B | G |
| Ivermectine (22,23-dihydroavermectines B₁ₐ /B_{1b}) | | **0,08** | **0,32** | **0,08** |
| Closantel* | | **4.0** | **16,0** | **4,0** |
| Agent anti-mottant (Silice colloïdale) | | **1,58** | | **-** |
| Agent anti-oxydant (BHA/BHT) | | **0,03** | **0,03** | **0,03** |
| Polyvinylpyrrolidone | | | **4,0** | |
| Agent conservateur (Alcool benzylique) | | **1,00** | | **1,00** |
| Huile de soja | q.s. | **100 ml** | | **-** |
| Propylène Glycol | q.s. | | **100 ml** | |
| Glycérides polyoxyéthylénes glycosés | q.s. | | | **100 ml** |

| | | | | |
|---|---|---|---|---|
| * Le closantel est introduit sous la forme de sel sodique en quantité suffisante pour garantir une teneur en closantel de 4% (p/V) dans la composition finale. | | | | |

**Tableau VIII : Mesures comparatives de la stabilité des formulations dans le temps.**

| | T 0 mois | | | | T 4mois | | | | T 6 mois | | | |
|---|---|---|---|---|---|---|---|---|---|---|---|---|
| | 25°C | | 40°C | | 25°C | | 40°C | | 25°C | | 40°C | |
| | Clos | Iver | Clos | Iver | Clos | Iver | Clos | Iver | Clos | Iver | Clos | Iver |
| Composition A | **96,15** | **100,2** | **96,15** | **100,2** | **96,27** | **100,2** | **99,6** | **101,9** | **95,0** | **99,67** | **97,4** | **99,8** |
| Composition B | **95,75** | **97,32** | **95,75** | **97,32** | **96,17** | **97,16** | **90,94** | **81,53** | **94,40** | **96,74** | **ND** | **ND** |
| Composition G | **95,02** | **102,7** | **95,02** | **102,7** | **90,19** | **100,3** | **84,36** | **98,41** | **86,54** | **97,96** | **ND** | **ND** |

On dose la quantité de principes actifs (ivermectine et closantel) dans les compositions et on calcule le pourcentage de matière active restante par rapport à la quantité introduite. Le résultat est exposé dans le tableau VIII. On constate qu'après 6 mois à 25°C la composition A comporte toujours plus de 98,8% de closantel par rapport à la quantité introduite initialement et que la composition G en revanche n'en comporte plus que 91%. A 40°C la stabilité du closantel dans la composition A est de 100%. On constate donc une différence significative de la stabilité du closantel entre les compositions A et G. Les stabilités de l'ivermectine et du closantel engagés dans une composition huileuse A, dans laquelle l'ivermectine est en solution et le closantel est en suspension conformément à l'invention sont parfaitement établies comparativement à la composition G dans laquelle l'ivermectine est en solution et le closantel est en solution et comparativement à la solution B qui comprend un solvant organique.

Il est reconnu que la démonstration de 6 mois de stabilité à une température de 40° Celsius équivaut à une stabilité en conditions ambiantes de 24 mois (2 ans).

### EXEMPLE 3

Composition huileuse conforme à l'invention pour administration par voie orale :
- Doramectine 0,06 % (p/V)
- Closantel (sel sodique) 4,0 % (p/V)
- agent anti-mottant (Silice colloïdale) 1,0 % (p/V)
- agent anti-oxydant (BHT) 0,1 % (p/V)
- Oléate d'éthyle 22,0 % (p/V)
- Huile de sésame q.s pour 100,0 ml.

### EXEMPLE 4

Composition huileuse conforme à l'invention, pour administration par voie orale :
- Eprinomectine 0,06 % (p/V)
- Closantel (sel sodique) 4,0 % (p/V)
- agent anti-mottant (Silice colloïdale) 1,0 % (p/V)
- agent anti-oxydant (BHT) 0,1 % (p/V)
- Triglycérides capriques/capryliques q.s pour 100,0 ml.

## Revendications

1. Composition huileuse **caractérisée en ce qu'**elle comprend :
- un véhicule huileux,
- au moins un composé choisi parmi les lactones macro cycliques, en solution dans le véhicule huileux,
- au moins un composé choisi parmi le closantel ou l'un de ses sels, en suspension dans ledit véhicule huileux.

2. Composition selon la revendication 1, **caractérisée en ce qu'**elle présente une stabilité des principes actifs pendant 2 ans d'au moins 90 % et préférentiellement d'au moins 95 %.

3. Composition selon l'une quelconque des revendications 1 à 2, **caractérisée en ce que** le véhicule est choisi pour convenir à une administration orale.

4. Composition antiparasitaire selon l'une quelconque des revendications 1 à 3, **caractérisée en ce que** le rapport pondéral entre les lactones macro cycliques et le closantel et/ou ses sels est compris entre 1 : 200 et 1 : 10.

5. Composition selon la revendication 4, **caractérisée en ce que** le rapport pondéral entre les lactones macro cycliques et le closantel et/ou ses sels est compris entre 1 : 100 et 1 : 50

6. Composition selon l'une quelconque des revendications 1 à 5, **caractérisée en ce que** la lactone macro cyclique est choisie dans le groupe constitué des composés LL-F28249, des milbémycines et des avermectines.

7. Composition selon la revendication 6, **caractérisée en ce que** la lactone macro cyclique est choisie dans le groupe constitué de l'ivermectine, l'abamectine, l'eprinomectine, la doramectine, la selamectine, la milbémycine oxime, la moxidectine.

8. Composition selon l'une quelconque des revendications 1 à 7, **caractérisées en ce que** le closantel est présent sous forme de sel sodique.

9. Composition selon l'une quelconque des revendications 1 à 8, **caractérisée en ce que** la taille des particules de closantel ou de ses sels est comprise entre 0,01 et 100 µm.

10. Composition selon la revendication 9, **caractérisée en ce que** la taille des particules de closantel ou de ses sels est comprise entre 0,1 et 20 µm.

11. Composition selon l'une quelconque des revendications 1 à 10, **caractérisée en ce que** le véhicule huileux est choisi parmi les huiles minérales, les huiles végétales, les huiles végétales semi-synthétiques et leurs mélanges.

12. Composition selon l'une quelconque des revendications 1 à 11, **caractérisée en ce en ce que** le véhicule huileux est choisi parmi les huiles végétales.

13. Composition selon la revendication 12, **caractérisée en ce que** le véhicule huileux est l'huile de soja.

14. Composition selon l'une quelconque des revendications 1 à 13, **caractérisée en ce qu**'elle comprend en outre au moins l'un des composés suivants : agents épaississants, agents anti-mottants, agents anti-agrégants, agents adsorbants, agents solubilisants, agents anti-oxydants, agents chélatants, agents conservateurs antimicrobiens, antibactériens, antifongiques.

15. Composition selon l'une quelconque des revendications 1 à 14, **caractérisée en ce qu'**elle comprend
- entre 0,01 et 0,48 %, en poids par rapport au poids total de la composition, d'au moins une lactone macro cyclique,
- entre 1 et 24 %, en poids par rapport au poids total de la composition, de closantel ou de l'un de ses sels.
- un véhicule huileux choisi parmi les huiles végétales, semi-synthétiques ou minérales.

16. Composition selon l'une quelconque des revendications 1 à 15, **caractérisée en ce qu'**elle comprend
- entre 0,04 et 0,08 %, en poids par rapport au poids total de la composition, d'avermectines B₁ ou 22,23-dihydroavermectines B₁ (ivermectines),
- environ 4 %, en poids par rapport au poids total de la composition, de closantel sodique.
- de l'huile de soja.

17. Composition selon l'une quelconque des revendications 1 à 16, pour son utilisation comme médicament.

18. Utilisation d'une composition selon l'une quelconque des revendications 1 à 17, pour la préparation d'un médicament destiné à la prévention et/ou au traitement des endo et/ou des ectoparasites.

19. Utilisation d'une composition selon l'une quelconque des revendications 1 à 17, pour la préparation d'un médicament destiné à la prévention et/ou au traitement d'un parasite choisi parmi les Plathelminthes et les Némathelminthes et les Arthropodes.

## Claims

1. Oily composition, **characterized in that** it comprises:
- an oily vehicle,
- at least one compound selected from macrocyclic lactones, in solution in the oily vehicle, and
- at least one compound selected from closantel and one of its salts, in suspension in said oily vehicle.

2. Composition according to Claim 1, **characterized in that** its active principles are at least 90% stable, preferably at least 95% stable, for 2 years.

3. Composition according to Claim 1 or 2, **characterized in that** the vehicle is selected to be suitable for oral administration.

4. Antiparasitic composition according to any one of Claims 1 to 3, **characterized in that** the weight ratio of the macrocyclic lactones to the closantel and/or its salts is between 1:200 and 1:10.

5. Composition according to Claim 4, **characterized in that** the weight ratio of the macrocyclic lactones to the closantel and/or its salts is between 1:100 and 1:50.

6. Composition according to any one of Claims 1 to 5, **characterized in that** the macrocyclic lactone is selected from the group comprising LL-F28249 compounds, milbemycins and avermectins.

7. Composition according to Claim 6, **characterized in that** the macrocyclic lactone is selected from the group comprising ivermectin, abamectin, eprinomectin, doramectin, selamectin, milbemycin oxime and moxidectin.

8. Composition according to any one of Claims 1 to 7, **characterized in that** the closantel is present in the form of the sodium salt.

9. Composition according to any one of Claims 1 to 8, **characterized in that** the particle size of the closantel or its salts is between 0.01 and 100 µm.

10. Composition according to Claim 9, **characterized in that** the particle size of the closantel or its salts is between 0.1 and 20 µm.

11. Composition according to any one of Claims 1 to 10, **characterized in that** the oily vehicle is selected from mineral oils, vegetable oils, semisynthetic vegetable oils and mixtures thereof.

12. Composition according to any one of Claims 1 to 11, **characterized in that** the oily vehicle is selected from vegetable oils.

13. Composition according to Claim 12, **characterized in that** the oily vehicle is soya bean oil.

14. Composition according to any one of Claims 1 to 13, **characterized in that** it also comprises at least one of the following compounds: thickeners, anticaking agents, antiaggregating agents, adsorbents, solubilizers, antioxidants, chelating agents and antimicrobial, antibacterial and antifungal preservatives.

15. Composition according to any one of Claims 1 to 14, **characterized in that** it comprises:
- between 0.01 and 0.48% by weight, based on the total weight of the composition, of at least one macrocyclic lactone,
- between 1 and 24% by weight, based on the total weight of the composition, of closantel or one of its salts, and
- an oily vehicle selected from vegetable, semisynthetic and mineral oils.

16. Composition according to any one of Claims 1 to 15, **characterized in that** it comprises:
- between 0.04 and 0.08% by weight, based on the total weight of the composition, of avermectins B₁ or 22,23-dihydroavermectins B₁ (ivermectins),
- about 4% by weight, based on the total weight of the composition, of closantel sodium, and
- soya bean oil.

17. Composition according to any one of Claims 1 to 16 for its use as a drug.

18. Use of a composition according to any one of Claims 1 to 17 for the preparation of a drug for the prevention and/or treatment of endoparasites and/or ectoparasites.

19. Use of a composition according to any one of Claims 1 to 17 for the preparation of a drug for the prevention and/or treatment of a parasite selected from Platyhelminthes, Nemathelminthes and Arthropodae.

## Patentansprüche

1. Ölige Zusammensetzung, **dadurch gekennzeichnet, dass** sie umfasst:
- ein öliges Vehikel,
- wenigstens eine Verbindung, ausgewählt aus makrozyklischen Laktonen, in Lösung im öligen Vehikel,
- wenigstens eine Verbindung, ausgewählt aus Closantel oder einem seiner Salze, in Suspension in dem öligen Vehikel.

2. Zusammensetzung nach Anspruch 1, **dadurch gekennzeichnet, dass** sie eine Stabilität der wirksamen Prinzipien während zwei Jahren von wenigstens 90% und vorzugsweise wenigstens 95% aufweist.

3. Zusammensetzung nach einem der Ansprüche 1 bis 2, **dadurch gekennzeichnet, dass** das Vehikel so ausgewählt ist, dass es zu einer oralen Verabreichung geeignet ist.

4. Antiparasitäre Zusammensetzung nach einem der Ansprüche 1 bis 3, **dadurch gekennzeichnet, dass** das Gewichtsverhältnis zwischen den makrozyklischen Laktonen und dem Closantel und/oder seinen Salzen zwischen 1:200 und 1:10 liegt.

5. Zusammensetzung nach Anspruch 4, **dadurch gekennzeichnet, dass** das Gewichtsverhältnis zwischen den makrozyklischen Laktonen und dem Closantel und/oder seinen Salzen zwischen 1:100 und 1:50 liegt.

6. Zusammensetzung nach einem der Ansprüche 1 bis 5, **dadurch gekennzeichnet, dass** das makrozyklische Lakton aus der Gruppe, gebildet durch die Verbindungen LL-F28249, Milbemycine und Avermectine, ausgewählt ist.

7. Zusammensetzung nach Anspruch 6, **dadurch gekennzeichnet, dass** das makrozyklische Lakton aus der Gruppe, gebildet von Ivermectin, Abamectin, Eprinomectin, Doramectin, Selamectin, Milbemycinoxim, Moxidectin, ausgewählt ist.

8. Zusammensetzung nach einem der Ansprüche 1 bis 7, **dadurch gekennzeichnet, dass** das Closantel in Form von Natriumsalz vorliegt.

9. Zusammensetzung nach einem der Ansprüche 1 bis 8, **dadurch gekennzeichnet, dass** die Größe der Partikel von Closantel oder von seinen Salzen zwischen 0,01 und 100 µm liegt.

10. Zusammensetzung nach Anspruch 9, **dadurch gekennzeichnet, dass** die Größe der Partikel von Closantel oder von seinen Salzen zwischen 0,1 und 20 µm liegt.

11. Zusammensetzung nach einem der Ansprüche 1 bis 10, **dadurch gekennzeichnet, dass** das ölige Vehikel aus Mineralölen, Pflanzenölen, semisynthetischen Pflanzenölen und ihren Gemischen ausgewählt ist.

12. Zusammensetzung nach einem der Ansprüche 1 bis 11, **dadurch gekennzeichnet, dass** das ölige Vehikel aus Pflanzenölen ausgewählt ist.

13. Zusammensetzung nach Anspruch 12, **dadurch gekennzeichnet, dass** das ölige Vehikel Sojaöl ist.

14. Zusammensetzung nach einem der Ansprüche 1 bis 13, **dadurch gekennzeichnet, dass** sie außerdem wenigstens eine der folgenden Verbindungen umfasst: Verdickungsmittel, Mittel, die die Klumpenbildung verhindern, Mittel, die die Aggregatbildung verhindern, Adsorptionsmittel, Solubilisierungsmittel, Antioxidantien, Chelatbildner, antimikrobielle, antibakterielle, antifungale Konservierungsmittel.

15. Zusammensetzung nach einem der Ansprüche 1 bis 14, **dadurch gekennzeichnet, dass** sie umfasst:
- zwischen 0,01 und 0,48 Gew.-%, bezogen auf das Gesamtgewicht der Zusammensetzung, an wenigstens einem makrozyklischen Lakton,
- zwischen 1 und 24 Gew.-%, bezogen auf das Gesamtgewicht der Zusammensetzung, an Closantel oder an einem seiner Salze,
- ein öliges Vehikel, ausgewählt aus pflanzlichen, semisynthetischen oder mineralischen Ölen.

16. Zusammensetzung nach einem der Ansprüche 1 bis 15, **dadurch gekennzeichnet, dass** sie umfasst:
- zwischen 0,04 und 0,08 Gew.-%, bezogen auf das Gesamtgewicht der Zusammensetzung, an Avermectinen B₁ oder 22,23-Dihydroavermectinen B₁ (Ivermectine),
- etwa 4 Gew.-%, bezogen auf das Gesamtgewicht der Zusammensetzung, an Closantel-Natrium,
- Sojaöl.

17. Zusammensetzung nach einem der Ansprüche 1 bis 16 zur Verwendung als Medikament.

18. Verwendung einer Zusammensetzung nach einem der Ansprüche 1 bis 17 zur Herstellung eines Medikaments, das für die Verhütung und/oder Behandlung von Endo- und/oder Ektoparasiten bestimmt ist.

19. Verwendung einer Zusammensetzung nach einem der Ansprüche 1 bis 17 zur Herstellung eines Medikaments, das für die Verhütung und/oder Behandlung eines Parasiten, ausgewählt aus Plathelminthen und Nemathelminthen und Arthropoden, bestimmt ist.
